# FASCICULE DE BREVET EUROPEEN

(11) **EP 0 635 491 B1**
(45) Date de publication et mention de la délivrance du brevet: **07.05.1997**
(21) Numéro de dépôt: 94401662.5
(22) Date de dépôt: 20.07.1994
(51) Int. Cl.: C07D 209/94, C07D 209/80, C07D 209/70, C07D 495/04, A61K 31/40

(54) **(Thia)Cycloalkyl(b)indoles, leur procédé de préparation et les compositions pharmaceutiques qui les contiennent**
(Thia)cycloalkyl(b)indole, Verfahren zu ihrer Herstellung und diese enthaltende Zusammensetzungen
(Thia) cycloalkyl(b)indoles, process of their preparation and compositions containing it

(30) Priorité: 20.07.1993 FR 9308862; 19.05.1994 FR 9406098
(43) Date de publication de la demande: 25.01.1995
(73) Titulaire: ADIR ET COMPAGNIE, 92415 Courbevoie Cédex (FR)
(72) Inventeur: Caubere, Paul, F-54000 Nancy (FR); Jamart-Gregoire, Brigitte, F-54500 Vandoeuvre les Nancy (FR); Caubere, Catherine, F-54000 Nancy (FR); Bizot-Espiard, F-75015 Paris (FR); Renard, Pierre, F-78000 Versailles (FR); Adam, Gérard, F-78600 Le Mesnil le Roi (FR)

(56) Documents cités:
- EP-A- 0 035 259
- EP-A- 0 404 536
- EP-A- 0 409 410
- EP-A- 1 511 169
- FR-A- 2 100 677
- US-A- 3 824 234
- US-A- 3 862 953

## Description

La présente invention concerne des (thia)cycloalkyl[b]indoles, leur procédé de préparation et les compositions pharmaceutiques qui les contiennent.

Les brevets EP-A-404536 et EP-A-409410 décrivent les indéno-indoles à propriété anti-oxydante.

Les brevets EP-A-35259, FR-A-2100677, US-A-3,862,953 et GB-A-151169 décrivent des (thia)cycloalkyl[b]indoles diversement substituées possédant entre autres des propriétés anti-inflammatoires. Enfin, certains cyclohept[b]indol-6-ones sont décrits dans le brevet US 3,824,234 comme antifongiques et analgésiques.

Il est désormais établi que la peroxydation lipidique est un facteur pathologique majeur. Notamment, il est clair que le processus de peroxydation lipidique et les produits qu'il génère peut être néfaste à la viabilité cellulaire.

Les effets de la peroxydation lipidique ont été impliqués dans de nombreuses conditions pathologiques telles que l'athérosclérose, les anémies hémolytiques et dommages dus à l'ischémie - reperfusion (oxidative Damage and repair, Chemical, Biological and Medical Aspects. 1991 Pergamon Press, page XXi). La possibilité de disposer de molécules permettant de lutter contre ce phénomène de peroxydation lipidique s'avère donc d'une grande utilité pour le clinicien pour la prévention et le traitement des pathologies impliquant un tel phénomène.

La demanderesse a découvert des nouveaux (thia)cycloalkyl[b]indoles possédant une action antioxydante puissante et permettant une protection des lipides, et notamment des LDL (Low Density Lipoproteins) humaines, contre l'oxydation.

L'invention concerne plus particulièrement les composés de formule (I) : 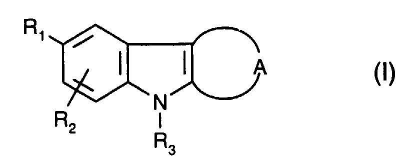 dans laquelle :
- A représente un chaîne alkylène -(CH₂)ₘ- avec m représentant 5 ou 6 ou un groupement de formule (α) 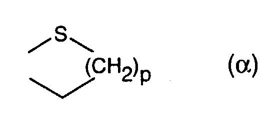 dans lequel p est compris entre 1 et 4,
- R₁ représente un radical choisi parmi :
   . hydroxy,
   . thiol,
   . O-R₄ avec R₄ étant choisi parmi alkyle et acyle ;
   . et -S-R₅ avec R₅ étant choisi parmi alkyle, phényle et benzyle ;
- R₂ représente un radical choisi parmi hydrogène, halogène, alkyle, alkoxy, et trifluorométhyle ;
- R₃ représente un radical choisi parmi alkyle, acyle, carboxyalkyle et alkoxycarbonylalkyle ; et, dans le cas où A est différent d'un pentaméthylène, R₃ peut également représenter un hydrogène,
avec la réserve que si m représente 5, R₂ représente un hydrogène et R₁ représente un méthoxy, alors R₃ ne peut pas représenter un méthyle,
leurs isomères optiques, sous forme pure ou sous forme de mélange, lorsque R₁, R₂ ou R₃ possèdent un centre chiral,
et leurs sels d'addition à une base pharmaceutiquement acceptable,
étant entendu que les termes "alkyle", "alkoxy" et "acyle" représentent des groupements linéaires ou ramifiés contenant de 1 à 6 atomes de carbone.

Parmi les bases pharmaceutiquement acceptables que l'on peut utiliser pour salifier les composés utilisés selon l'invention, on peut citer, à titre d'exemples et de façon non limitative, l'hydroxyde de sodium, l'hydroxyde de potassium, la triéthylamine, la diéthylamine, l'éthanolamine, l'arginine, la lysine, et la diéthanolamine.

L'invention concerne également le procédé de préparation des composés de formule (I) caractérisé en ce que l'on condense, en présence d'une base telle qu'un amidure alcalin ou sa combinaison avec un alcoolate alcalin (base complexe) et préférentiellemet la base complexe NaNH₂-t.BuONa le composé de formule (II) : 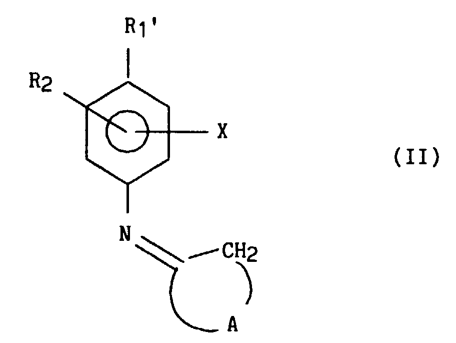 dans laquelle R₂ et A sont tels que définis dans la formule (I), R₁' est choisi parmi -O-R₄ et -S-R₅ avec R₄ et R₅ tels que définis dans la formule (I) et X représente un atome d'halogène, pour obtenir :
- soit un composé de formule (III) : 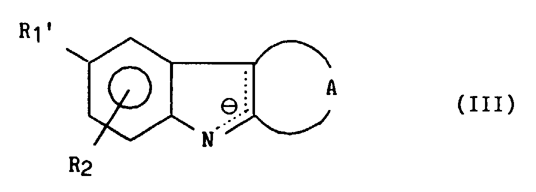 dans laquelle R₂, A et R₁' sont tels que définis précédemment,
- soit, après hydrolyse un composé de formule (Iₐ) : 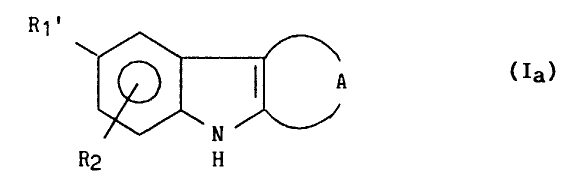 dans laquelle R₂, A et R₁' sont tels que définis précédemment,
   composé de formule (III), ou composé de formule (Iₐ) sous forme de son sel alcalin, qui est substitué sur l'azote indolique par un radical de formule R₃', avec R₃' ayant la même signification que R₃ tel que défini dans la formule (I) à l'exception de l'hydrogène, afin d'obtenir un composé de formule (Iₐ') : 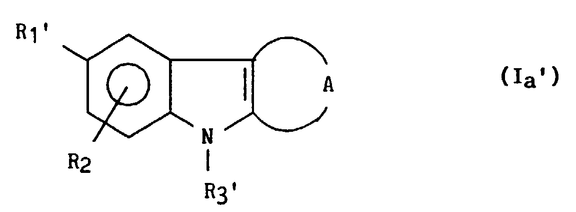 dans laquelle R₂, A, R₁' et R₃' sont tels que définis précédemment,
   composés de formule (Iₐ) ou (Iₐ') qui sont, si nécessaire,
- soit soumis à une réaction de désalkylation afin d'obtenir un composé de formule (I_{b}) : 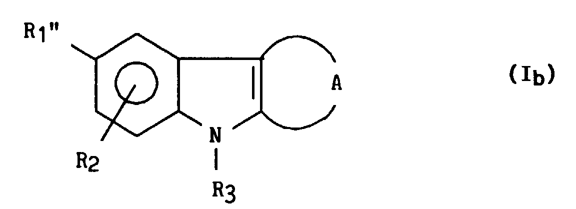 avec R₂, R₃ et A tels que définis précédemment et R₁'' représentant un groupement hydroxy ou thiol,
- soit soumis à l'action d'un mélange de formule (IV) :

   Al X'₃, R₅'SH (IV)

   dans laquelle X' représente un halogène et R₅' représente un alkyle, un phényle ou un benzyle,
pour obtenir, suivant les conditions d'utilisation du composé de formule (IV) :
* un composé de formule (I_{b}) tel que défini précédemment,
* ou un composé de formule (I_{c}) : 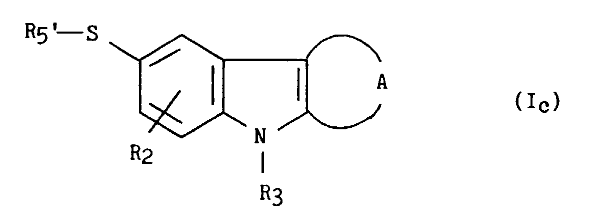 dans laquelle R₂, R₃, R₅' et A sont tels que définis précédemment,
   composé de formule (I_{c}) qui peuvent être soumis à une hydrogénation pour obtenir le composé de formule (I_{d}) : 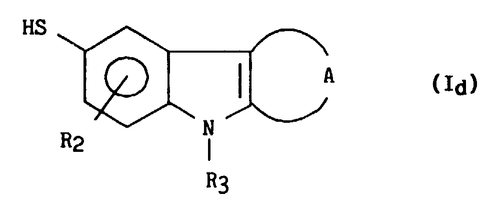 dans laquelle R₂, R₃ et A sont tels que définis précédemment,
   étant entendu que les termes "alkyle", "alkoxy" et "acyle" représentent des groupements linéaires ou ramifiés contenant de 1 à 6 atomes de carbone,
   les composés de formule (Iₐ), (Iₐ'), (I_{b}), (I_{c}) et (I_{d}) formant l'ensemble des composés de formule (I),
   les composés de formule (I) pouvant être, le cas échéant :
   - purifiés suivant une ou plusieurs méthodes choisies parmi la cristallisation, la chromatographie sur colonne de silice, l'extraction, la filtration, et le passage sur charbon ou résine,
   - séparés, sous forme pure ou sous forme de mélange, en leurs éventuels isomères optiques,
   - et salifiés par une base pharmaceutiquement acceptable.

La substitution du composé de formule (Iₐ) ou (Iₐ'), tel que définis précédemment, par un radical de formule -R₃', prévue dans le procédé de synthèse précédent, est réalisable par réaction d'un composé de formule (Iₐ) ou d'un sel alcalin d'un composé de formule (Iₐ') avec un composé de formule R₃'-X'', Cl-COO-R₃'', (R₃'-O)₂SO₂, X-(CH₂)ₙ-COOR₃'', ou (R₃''-O-CO)₂O dans lesquelles R₃' est tel que défini précédemment, R₃'' représente un radical (C₁-C₆) alkyle, n représente un entier de 1 à 6 et X" représente un halogène, réaction éventuellement suivie d'une étape d'hydrolyse pour l'obtention de la fonction acide à partir de l'ester, dans le cas ou R₃' représente un alkoxycarbonylalkyle.

PAR EXEMPLE L'INVENTION CONCERNE :
- la préparation de la base complexe :
   A une suspension de 7 équivalents (éq.) de NaNH₂ (dont 2 éq. pour la préparation de l'alcoolate et 1 éq. pour la préparation de l'énolate d'imine ou d'énamine) dans du tétrahydrofurane (THF) (7cm³ pour 70 mmoles de NaNH₂), on ajoute goutte à goutte 2 éq. de 2-méthyl-propan-2-ol à température ambiante. Après cet ajout, le mélange est chauffé à 45°C pendant 2 heures (le rapport NaNH₂/t-BuONa est alors de 2/1 lors de l'étape de cyclisation).
- la cyclisation des imines :
   Au milieu basique préparé ci-dessus, on ajoute à 0°C, 1 éq. de l'imine à condenser en solution dans le THF (3 cm³ pour 1 mmole). Le mélange est agité magnétiquement à température ambiante ou 40-45°C. La réaction est suivie par chromatographie en phase gazeuse.
- La préparation des indoles non substitués sur l'azote (hydrolyse) :
   Lorsque tout le produit de départ a disparu, on procède à une hydrolyse à 0°C. Après une extraction à l'éther, la phase organique est séchée sur MgSO₄ et les solvants évaporés sous pression réduite. Le (thia)cycloalkyl[b]indole est ensuite séparé par chromatographie en phase liquide.
- la N-méthylation in situ : préparation des thiacycloalkyl[b]indoles N-méthylés :
   Lorsque tout le produit de départ a disparu, le milieu réactionnel est décanté puis la partie liquide est transférée dans une ampoule de Mariotte puis ajoutée goutte à goutte à 3 éq. de Me₂SO₄ en solution dans du THF à 0°C. A la fin de l'addition, on laisse le milieu réactionnel remonter à température ambiante. Après 2 heures, le mélange est hydrolysé avec une solution aqueuse de NH₄OH à 32 % puis extrait au chlorure de méthylène. La phase organique est ensuite séchée sur MgSO₄ et les solvants évaporés sous pression réduite. Le (thia)cycloalkyl[b]indole N-méthylé est ensuite séparé par chromatographie en phase liquide.
- La N-carbéthoxyméthylation in situ : préparation des thiacycloalkyl[b] indoles N-carbéthoxyméthylés :
   La partie liquide du milieu réactionnel est transférée dans une ampoule à additionner et ajoutée goutte à goutte à une solution de BrCH₂COOEt (3 éq.) dans du diméthylformamide (DMF) (6cm³ pour 1 mmole à température ambiante). Après 2 heures, on extrait à l'éther, et la phase organique est lavée à l'eau puis séchée sur MgSO₄ et les solvants évaporés sous pression réduite. Le thiacycloalkyl[b]indole ainsi obtenu est ensuite séparé par chromatographie en phase liquide.
- la saponification : formation des N-(carboxyméthyl) thiacycloalkyl[b]indoles :
   Le N-(carbéthoxyméthyl) thiacycloalkyl[b]préparé ci-dessus est placé à reflux dans une solution à 10 % de KOH dans l'éthanol. La réaction est suivie par chromatographie sur couche mince (ccm).

lorsque tout le produit de départ a disparu, la réaction est jetée sur glace et extraite à l'éther. La phase aqueuse est alors acidifiée et extraite à nouveau à l'éther. La phase organique est séchée sur MgSO₄ puis les solvants sont évaporés sous pression réduite. Le N-(carboxyméthyl) thiacycloalkyl[b]est purifié par chromatographie en phase liquide.

Les composés de formule (II) sont aisément accessibles à l'homme du métier par réaction d'une aniline de formule (IIa) : 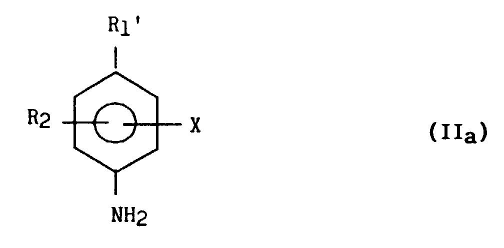 dans laquelle R₁' et R₂ sont tels que définis précédemment,
avec une cétone de formule (V) : 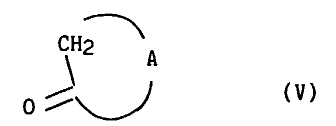 dans laquelle A est tel que décrit dans la formule (I).

### SYNTHESE D'IMINES ET ENAMINES

Plus particulièrement, les imines peuvent être préparées en soumettant 1 éq. d'halogénoamine et 1 éq. de cétone à une distillation azéotropique dans le benzène. (selon les cas la réaction peut être catalysée par un acide (APTS, ZnCl₂, ZnBr₂, BF₃-Et₂O)).
Après avoir récolté la quantité d'eau adéquate, l'imine est distillée sous pression réduite.

Les matières premières utilisées lors des procédés de préparation précédemment décrits sont soit commerciales, soit aisément accessibles à l'homme de l'art d'après la littérature.

Les composés de la présente invention possèdent des propriétés anti-oxydantes très importantes. Les études pharmacologiques ont notamment montré que ces composés sont doués d'activités protectrices remarquables et spécifiques vis-à-vis des peroxydations lipidiques et notamment de peroxydations des lipoprotéines de faible densité (LDL).

Les composés de l'invention présentent donc une action particulièrement nouvelle et bénéfique dans les affections où intervient un phénomène de peroxydation, notamment une peroxydation lipidique. Ces nouveaux composés peuvent être utilisés dans le traitement ou la prévention des affections dues ou reliées à de tels phénomènes de peroxydation et notamment les désordres ischémiques cérébraux, rénaux ou cardiaques, l'athérosclérose, les anémies hémolytiques, les dommages dus au processus de reperfusion, et les syndromes inflammatoires.

De par leur action, les composés de l'invention sont également de nouveaux candidats cliniques pour le traitement et la prévention des maladies inflammatoires et des conditions inflammatoires pathologiques.

Les composés de l'invention sont en effet de puissants inhibiteurs de la lipoxygénase (Etude pharmacologique : exemple A) et possèdent une intense activité anti-inflammatoire (Etude Pharmacologique : exemple B).

Les composés de l'invention sont par conséquent utiles dans le traitement et la prévention de l'inflammation chronique ou aiguë, articulaire, pulmonaire, cutanée, ou rénale et notamment dans la prévention et le traitement de l'arthrite, de la polyarthrite rhumatoïde, de l'ostéo-arthrose, du psoriasis, des maladies allergiques, de l'asthme, des maladies inflammatoires de l'intestin, des ulcères gastro-intestinaux,de l'ischémie, de l'athérosclérose, de la détresse respiratoire, et du choc septique.

Les composés de l'invention sont donc des antioxydants très puissants et possèdent également une forte activité antiinflammatoire.
Il s'avère que les composés de l'invention montrent également une activité antiagrégante (test d'inhibition de l'agrégation induite par l'acide arachidonique. BERTELE et al Science, 1983, 220 : pp 517-519), une activité hypolypémiante (mesure de l'hypocholestérolémie : Day et al, Atherosclerosis Drug Discovery, Edit. Charles E. Day, Plenum Publishing Corp. New York, 1976, pp 231-249 ; Holub et al, Clin. Chem, 1972, 18, pp 239-243 ; Allain et al, Clin. Chem, 1974, 20 (4), pp 470-475) ainsi qu'une activité bronchorelaxante (test de relaxation trachéale in vitro, Ludvena et al, Arch. Int. Pharmacodyn, 1957, 111, pp 392-400).

La présente invention a également pour objet les compositions pharmaceutiques contenant un composé de formule (I), ou un de ses sels d'addition à une base ou à un acide pharmaceutiquement acceptable, en combinaison avec un ou plusieurs excipients pharmaceutiquement acceptables.

Parmi les compositions pharmaceutiques selon l'invention, on pourra citer plus particulièrement celles qui conviennent à l'administration orale, percutanée, cutanée, parentérale, nasale, rectale, perlinguale, oculaire ou pulmonaire et notamment les préparations injectables ou buvables, les aérosols, les gouttes oculaires ou nasales, les comprimés simples, pelliculés ou dragéifiés, les gélules, les capsules, les crèmes, pommades, gels dermiques, les pilules, les paquets, les sachets, les granulés et les suppositoires.

La posologie varie selon l'âge, le poids et le sexe du patient, la voie d'administration, la nature et l'intensité de l'affection, et selon les éventuels traitements associés. Les doses s'échelonnent entre 0,5 mg à 2 g par jour, particulièrement entre 0,5 mg à 100 mg par jour, par exemple entre 10 mg à 100 mg par jour.

Les exemples qui suivent illustrent l'invention mais ne la limitent en aucune façon.

### Préparation 1 : N-CYCLOHEPTYLIDENYL-3-CHLORO-4-METHOXYANILINE

Le composé du titre est obtenu par distillation azéotropique à partir de la 3-chloro-4-méthoxyaniline et de la cycloheptanone, en utilisant l'acide paratoluènesulfonique comme catalyseur et le benzène comme solvant.
La réaction est suivie par chromatographie en phase gazeuse. Après achèvement de la réaction, le milieu est ramené à température ambiante, traité par une solution saturée en NaHCO₃, extrait par de l'éther et séché sur sulfate de magnésium. Les solvants sont éliminés sous vide.
L'imine est purifiée par distillation ou utilisée telle quelle.

### Préparation 2 : N-CYCLOOCTYLIDENYL-3-CHLORO-4-METHOXYANILINE

En procédant comme dans la préparation 1 mais en utilisant la cyclooctanone à la place de la cycloheptanone, on obtient le composé du titre.

### Préparation 3 : N-(thiopyraniliden-3-yl)-3-chloro-4-méthoxyaniline

1 éq. de 3-chloroparaanisidine et 1 éq. de thiopyran-3-one sont soumis à une distillation azéotropique dans du benzène (100 cm³ pour 50 mmoles). La réaction est suivie par chromatographie gazeuse et arrêtée au bout de 5 h. L'imine est purifiée par distillation et est obtenue avec un rendement de 40%.
RMN¹H : 6,3-7,0 (3H, m, arom. H) ; 3,7 (3H, s, OMe) ; 3,3 (1H, s, C-H) ; 3,1 (1H, s, C-H) ; 2,0-3,0 (m, 6H, 3 x CH₂)
Masse : 255

### EXEMPLE 1 : 3-HYDROXY-10-METHYL-5,6,7,8,9,10-HEXAHYDRO-CYCLOHEPT[b] INDOLE

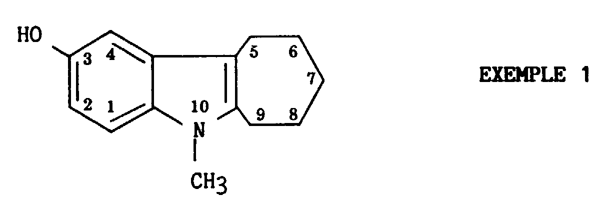

### STADE A : 3-méthoxy-5,6,7,8,9,10-hexahydro-cyclohept[b]indole

### Préparation de la base complexe :

A une suspension de 7 équivalents (ci-après abrégé en eq.) de NaNH₂ dans du tétrahydrofurane (THF) (7 cm³ pour 70 mmoles de NaNH₂), on ajoute goutte à goutte 2 eq. de 2-méthyl-propan-2-ol à température ambiante. Après cet ajout, le mélange est chauffé à 45°C pendant 2 h.

### Condensation :

On ajoute, à 0°C, 1 eq. de N-cycloheptylidènyl-3-chloro-4-méthoxyaniline à la base complexe préparée ci-dessus. Le mélange est agité à température ambiante jusqu'à achèvement de la réaction. La réaction est suivie par chromatographie gazeuse. Le 3-méthoxy-5,6,7,8,9,10-hexahydro-cyclohept [b]indole est séparé par chromatographie en phase liquide.

### STADE B : 3-méthoxy-10-méthyl-5,6,7,8,9,10-hexahydro-cyclohept [b]indole

Après décantation, le liquide obtenu ci-dessus est transféré dans une solution de 3 eq. de sulfate de diméthyle, sous agitation, à 0°C. Le milieu réactionnel est ensuite mis sous agitation pendant 1 h à température ambiante. Le mélange est versé sur de la glace, extrait par de l'éther, et lavé avec une solution d'hydroxyde d'ammonium à 32 %. Le composé attendu est purifié par chromatographie en phase liquide.
Rendement : 53 %

### 2ème procédé :

Le dérivé indolique obtenu au stade précédent, en solution dans le diméthylformamide (DMF) est ajouté goutte à goutte, à 0°C, à une suspension de 2 eq. d'hydrure de sodium dans du DMF (10 cm³ de DMF pour 1 mmole de substrat indolique).
On laisse le milieu réactionnel revenir à température ambiante puis on ajoute 3 eq. de sulfate de diméthyle.
Le milieu est ensuite traité de la même façon que dans le premier procédé ci-dessus.

### STADE C : 3-hydroxy-10-méthyl-5,6,7,8,9,10-hexahydro-cyclohept [b]indole

1 eq. du composé obtenu au stade précédent dilué dans du chlorure de méthylène (5 cm³ pour 3 mmoles) est ajouté, goutte à goutte, à 0°C à un mélange composé de 1,5 eq. de AlCl₃ et 20 eq. de CH₃CH₂-SH.
Rendement : 60 - 91 %
Point de fusion : 102°C
Microanalyse :

| | C % | H % | N % |
|---|---|---|---|
| Calculé (calc.) | 78.10 | 7.96 | 6.50 |
| Trouvé (Tr.) | 77.59 | 7.85 | 6.52 |

Caractéristiques spectrales :
IR (cm⁻¹) : 3349 (OH) ; 2920-2845 (aliphatique).
RMN¹H (CDCl₃) δ ppm : 6.5-7.3 (m, 3H, Aromatique H) 5.2(s, 1H, OH échangé avec D₂O) ; ; 3.6 (s, 3H, NCH₃) ; 2.5-3.0 (m, 4H, 2xCH₂) ; 1.5-2.0 (m, 6H, 3xCH₂).

### EXEMPLE 2 : 3-METHOXY-10-METHYL-5,6,7,8,9,10,11-HEPTAHYDRO-CYCLOOCT[b]INDOLE

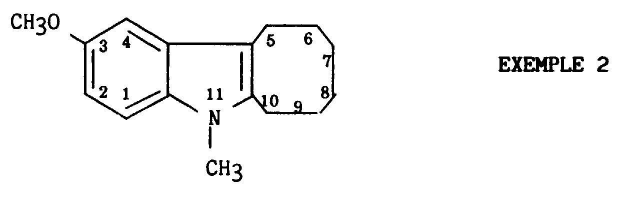

En procédant comme aux stades A et B de l'exemple 1, mais en remplaçant au stade A la N-cycloheptylidènyl-3-chloro-4-méthoxyaniline par la N-cyclooctylidènyl-3-chloro-4-méthoxyaniline, on obtient le composé du titre.
Rendement : 60 %
Point de fusion (éther de pétrole + éthanol) : 54°C
Microanalyse :

| | C % | H % | N % |
|---|---|---|---|
| Calculé | 78.96 | 8.69 | 5.75 |
| Trouvé | 78.90 | 8.52 | 5.82 |

Caractéristiques spectrales :
IR (cm⁻¹) : 2921-2848 (aliphatique).
RMN¹H (CDCl₃) δ ppm : 7.10-6.50 (m,3H,Aromatique H) ; 3.78 (s,3H,OCH₃) ; 3.68 (s,3H,NCH₃) ; 3.00-2.60(m,4H,2xCH₂) ; 1.90-1.20 (m, 8H,4xCH₂).

### EXEMPLE 3 : 3-ETHYLTHIO-10-METHYL-5,6,7,8,9,10-HEXAHYDROCYCLOHEPT[b] INDOLE

A un mélange refroidi à 0°C de 380 mg (2,84 mmol) de chlorure d'aluminium et de 2,3 g (37,9 mmol) d'éthanethiol, sont ajoutés goutte à goutte 500 mg (1,9 mmol) du composé obtenu au stade B à l'exemple 1, préalablement dissous dans 5 cm³ de chlorure de méthylène distillé sur pentoxyde de phosphore. Après 1 h d'agitation à 0°C sont ajoutés à nouveau 1,5 éq. de chlorure d'aluminium et 20 éq. d'éthanethiol. Le mélange est agité encore 1 h à 0°C puis versé sur glace, traité par de l'acide chlorhydrique 1N, puis extrait au dichlorométhane. Après traitement de la phase organique et purification du produit brut par chromatographie sur colonne de silice, le composé attendu est obtenu.
Rendement : 55 %
Point de fusion : 44°C

| | C % | H % | N % | S % |
|---|---|---|---|---|
| Calculé | 74.07 | 8.16 | 5.39 | 12.36 |
| Trouvé | 74.04 | 8.16 | 5.36 | 12.40 |

IR (cm⁻¹) : 2921-2846(aliphatique)
RMN¹H (CDCl₃) δ ppm : 7.60 (s, 1H, Aromatique H) 7.25-7.10 (m, 2H, Aromatique H) ; 3.60 (s, 3H, NCH₃) ; 2.90-2.70 (q+m, 6H, SCH₂ + 2xCH₂) ; 1.95-1.70 (m, 6H, 3xCH₂) ; 1.30-1.15 (t, 3H, CH₃).
RMN¹³C (CDCl₃) δ ppm : 139.86 (C5) ; 135.17 (C1) ; 128.25 (C3) ; 125.16 (C4) ; 123.77 (C8) ; 122.37 (C6) ; 111.32 (C2) ; 109.10 (C7) ; 31.46-30.81 (C9) ; 29.55 (C16)-28.31-26.93-26.25-24.22 (C11,12,13,14,15) ; 14.80 (C10).

### EXEMPLE 4 : 2-[3-METHOXY-5,6,7,8,9,10-HEXAHYDRO-CYCLOHEPT[b]INDOL-10-YL]ACETATE D'ETHYLE

En procédant comme dans l'exemple 1, aux stades A et B mais en utilisant, au stade B, le bromoacétate d'éthyle au lieu du sulfate de diméthyle, on obtient le composé du titre.

### EXEMPLE 5 : ACIDE 2-[3-METHOXY-5,6,7,8,9,10-HEXAHYDRO-CYCLOHEPT [b]INDOL-10-YL]ACETIQUE

En réalisant l'hydrolyse de la fonction ester du composé obtenu à l'exemple 4, on obtient le composé du titre.

### EXEMPLE 6 : 10-METHYL-3-PHENYLTHIO-5,6,7,8,9,10-HEXAHYDRO-CYCLOHEPT [b]INDOLE

En procédant comme dans l'exemple 3, mais en remplaçant au stade C l'éthanethiol (CH₃CH₂-SH) par le thiophénol (C₆H₅-SH), on obtient le composé du titre.

### EXEMPLES 7 A 21 :

En procédant selon les méthodes décrites dans les exemples précédents et en utilisant les réactifs appropriés, on peut obtenir les composés des exemples exposés dans le tableau (I) suivant :

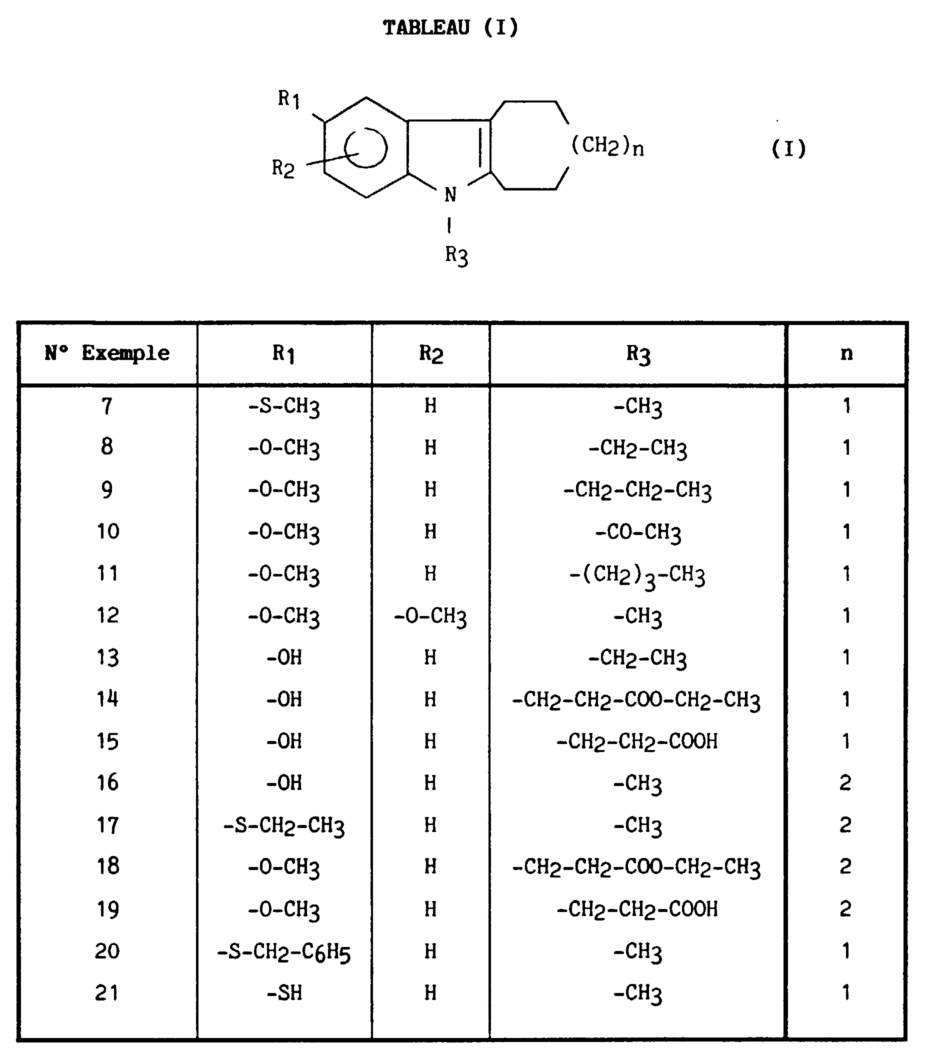

Données physico-chimiques :
Point de fusion de l'exemple 16 : 98 °C

### - Synthèse des thiopyrano[3,2-b]indoles par cyclisation d'imines suivie d'une hydrolyse :

La synthèse d'indole est décrite pour le composé de l'exemple 22 :

### EXEMPLE 22 : 8-METHOXY-THIOPYRANO[3,2-b]INDOLE

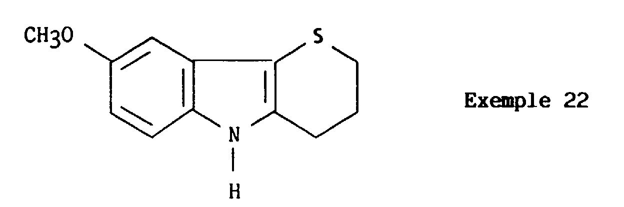

### STADE A :

### Préparation de la base complexe :

A une suspension de 7 éq. de NaNH₂ dans du tétrahydrofurane (7 cm³ pour 70 mmoles de NaNH₂), on ajoute goutte à goutte 2 éq. de 2-méthyl-propan-2-ol à température ambiante. Après cet ajout, le mélange est chauffé à 45°C pendant 2 heures.

### STADE B :

### Condensation :

### Procédure 1 :

A la base complexe préparée ci-dessus, on ajoute à 0°C, 1 éq. de N-(thiopyraniliden-3-yl)-3-chloro-4-méthoxyaniline (préparation 3) en solution dans du THF (30 cm³ pour 10 mmoles).

Le mélange est agité à température ambiante pendant 12 h. La réaction est suivie par chromatographie gazeuse. A la fin de la réaction, le milieu réactionnel est jeté sur glace et extrait à l'éther.

Après séchage sur MgSO₄ et évaporation des solvants sous pression réduite, le composé du titre est isolé par chromatographie éclair (Kieselgel 40-63µ) avec un éluant 5 % Acétate d'éthyle/éther de pétrole.
Rendement : 53 % par rapport à l'imine
Point de fusion (Totoli) : 123°C
IR : 3390 (NH), 2999, 2938, 2922, 2834 (C-H)

| | | | | | |
|---|---|---|---|---|---|
| Analyse (C₁₂H₁₃ONS) : | Calc. : | C 65,72 | H 5,97 | N 6,38 | S 14,62 |
| | Tr. : | 65,95 | 6,03 | 6,53 | 14,93 |

### Procédure 2 :

L'imine brute préparée lors de la préparation 3 à partir de 1 éq. de cétone + 1 éq. d'amine, est ajoutée à 0°C, sans purification, à la base complexe préparée ci-dessus. La réaction est effectuée comme pour la procédure 1.
Rendement : 36, 5 % par rapport à la cétone.

### - Synthèse d'indoles N-carbéthoxyméthylés :

La synthèse sera décrite pour le composé de l'exemple 23 :

### EXEMPLE 23 : 2-(8-METHOXY-THIOPYRANO[3,2-b]INDOL-5-YL) ACETATE D'ETHYLE

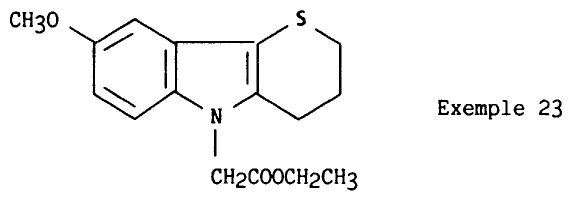

A la fin de la réaction effectuée dans l'exemple 22 (procédure 1 ou 2), le milieu réactionnel est laissé décanté sous courant d'azote. Le surnageant est transféré à température ambiante dans une solution de 4 éq. de BrCH2COOCH₂CH₃ dans du diméthylformamide (DMF) (de façon à avoir un mélange THF/DMF = 1/2 à la fin de l'addition). Lorsque la réaction est terminée (suivie par chromatographie sur couche mince (ccm)), le milieu réactionnel est jeté sur glace, extrait à l'éther. Après séchage sur MgSO₄ et évaporation des solvants, le composé du titre est isolé par chromatographie éclair avec un éluant 15 % acétone/hexane.
Rendement : 46 % par rapport à l'imine
Point de fusion (Totoli) : 98 °C
IR : 2923 (C-H) ; 1750 (C=0)

| | | | | | |
|---|---|---|---|---|---|
| Analyse (C₁₆H₁₉O₃NS) : | Calc. : | C 62,92 | H 6,27 | N 4,58 | S 10,49 |
| | Tr. : | 62,58 | 6,32 | 4,69 | 10,36 |

### EXEMPLE 24 : 8-METHOXY-5-METHYL-THIOPYRANO[3,2-b]INDOLE

Le composé obtenu dans l'exemple 22 est soumis à l'action de 3 éq. de sulfate de diméthyle, sous agitation, à 0°C. Le milieu réactionnel est ensuite mis sous agitation pendant 1 h à température ambiante. Le mélange est versé sur de la glace, lavé avec une solution d'hydroxyde d'ammonium à 32 % puis extrait par de l'éther. Le composé attendu est purifié par chromatographie en phase liquide.
Rendement : 53 %

### 2 ème procédé :

Le composé obtenu à l'exemple 22, en solution dans du diméthylformamide (DMF) est ajouté goutte à goutte, à 0°C, à une suspension de 2 éq. d'hydrure de sodium dans du DMF (10 cm³ de DMF pour 1 mmole de substrat indolique). On laisse le milieu réactionnel revenir à température ambiante puis on ajoute 3 éq. de sulfate de diméthyle. Le milieu est ensuite traité de la même façon que dans le premier procédé ci-dessus.

### EXEMPLE 25 : 8-HYDROXY-5-METHYL-THIOPYRANO[3,2-b]INDOLE

Le composé de l'exemple 25 est obtenu par déméthylation du composé obtenu à l'exemple 24.

### EXEMPLE 26 : 8-ETHYLTHIO-5-METHYL-THIOPYRANO[3,2-b]INDOLE

A un mélange refroidi à 0°C de 380 mg (2,84 mmol) de chlorure d'aluminium et de 2,3 g (37,9 mmol) d'éthanethiol, sont ajoutés goutte à goutte 500 mg (1,9 mmol) du composé obtenu à l'exemple 24, préalablement dissous dans 5 cm³ de chlorure de méthylène distillé sur pentoxyde de phosphore. Après 1h d'agitation à 0°C sont ajoutés à nouveau 1,5 éq. de chlorure d'aluminium et 20 éq. d'éthanethiol. Le mélange est agité encore 1 h à 0°C puis versé sur glace, traité par de l'acide chlorhydrique 1 N, puis extrait au dichlorométhane. Après traitement de la phase organique et purification du produit brut par chromatographie sur colonne de silice, le composé attendu est obtenu.

### EXEMPLE 27 : ACIDE 2-(8-METHOXY-THIOPYRANO[3,2-b]INDOL-5-YL)ACETIQUE

En réalisant l'hydrolyse de la fonction ester du composé obtenu à l'exemple 23, on obtient le composé du titre.

### EXEMPLE 28 : 5-METHYL-8-BENZYLTHIO-THIOPYRANO[3,2-b]INDOLE

En procédant comme dans l'exemple 26, mais en remplaçant l'éthanethiol (CH₃CH₂SH) par le benzylthiol (C₆H₅-CH₂-SH), on obtient le composé du titre.

### EXEMPLES 27 A 36 :

En procédant selon les méthodes décrites dans les exemples précédents et en utilisant les réactifs appropriés, on peut obtenir les composés exposés dans le tableau (II) suivant :

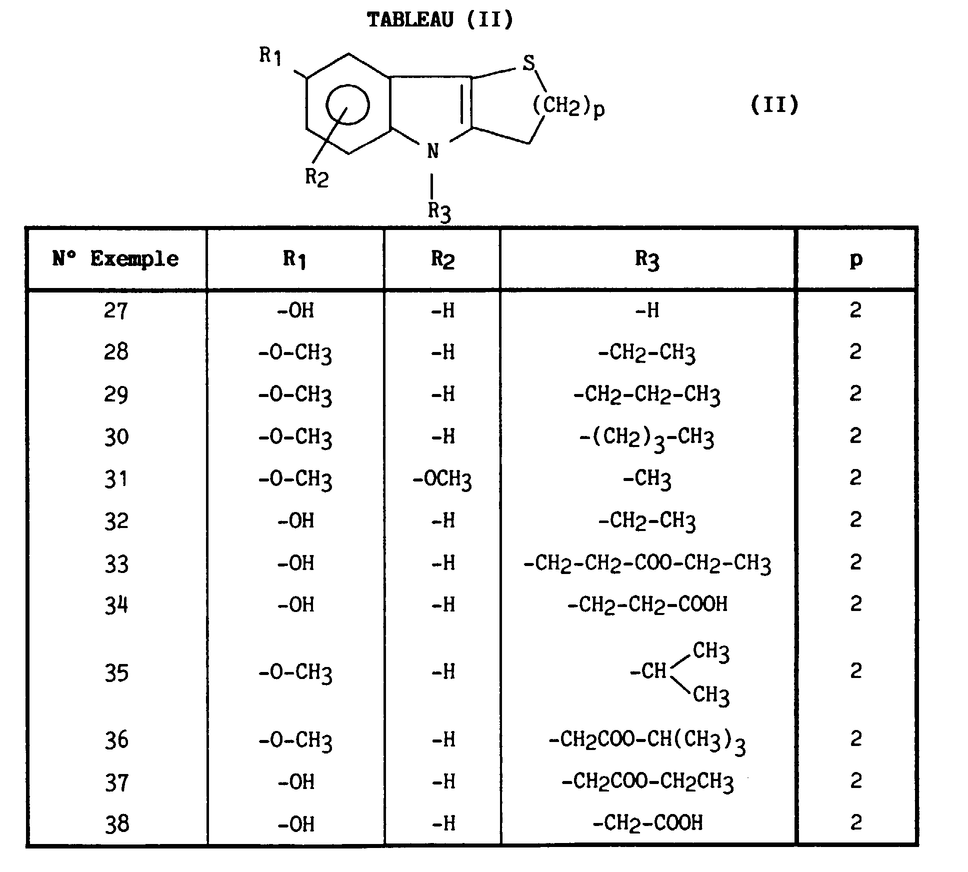

### EXEMPLE 39 :2-[3-HYDROXY-5,6,7,8,9,10,11-HEPTAHYDROCYCLOOCT[b]INDOL-11-YL]ACETATE D'ETHYLE

Point de fusion : 100°C

### EXEMPLE 40 : ACIDE 2-[3-HYDROXY-5,6,7,8,9,10,11-HEPTAHYDROCYCLOOCT[b]INDOL-11-YL]ACETIQUE

### EXEMPLE 41 : 3-METHOXY-5,6,7,8,9,10,11-HEPTAHYDROCYCLOOCT[b]INDOLE

### EXEMPLE 42 : 2-[3-METHOXY-5,6,7,8,9,10,11-HEPTAHYDROCYCLOOCT[b]INDOL-11-YL]ACETATE D'ETHYLE

### EXEMPLE 43 :2-[3-METHOXY-5,6,7,8,9,10,11-HEPTAHYDROCYCLOOCT[b]INDOL-11-YL]ACETATE DE TERT.BUTYLE

### ETUDE PHARMACOLOGIQUE

### EXEMPLE A : ETUDE DU POUVOIR PROTECTEUR DE L'OXYDATION DES LDL

La capacité des composés de l'invention à diminuer les proportions de LDL oxydées a été mesurée de la façon suivante : on réalise une incubation de 24 h regroupant des LDL (Low Density Lipoproteins) natives, un système Cu²⁺ générateur de radicaux libres et les composés à tester.

Les résultats sont obtenus après analyse du milieu par une technique chromatographique haute performance : la FPLC (Fast Protein Liquid Chromatography). Le pouvoir protecteur du composé testé est déterminé après comparaison du chromatogramme obtenu avec celui du témoin positif de référence : le probucol. Il apparaît clairement que les composés de l'invention ont un pouvoir protecteur très important. A titre de comparaison, pour une concentration de 10⁻⁵ M, le niveau de protection obtenu pour les composés de l'invention dépasse celui du probucol. C'est le cas notamment du composé de l'exemple 1 qui permet une très forte protection vis-à-vis d'un système oxydant des LDL (75 % de forme non oxydée à 10⁻⁵ M).

### EXEMPLE B : ETUDE DE L'ACTIVITE ANTIPEROXYDANTE

L'action des composés utilisés selon l'invention, susceptibles de piéger les radicaux HO., a été étudiée d'une part, sur la peroxydation spontanée des lipides et d'autre part, sur la peroxydation induite par le système Fe²⁺ - ascorbate (10 µM - 250 µM), et ce, sur des homogénats de cervaux de rats.
a) Etude de la peroxydation lipidique spontanée
   Lors de la mesure de la peroxydation lipidique spontanée, les homogénats de cerveau de rats sont placés en présence ou en absence des composés à tester durant 60 min à 37°C. La réaction est arrêtée à 0°C et le dosage du malondialdéhyde est effectué à l'aide d'acide thiobarbiturique. La peroxydation lipidique est déterminée par les substances réagissant avec l'acide thiobarbiturique exprimées en nanomoles de malondialdéhyde.
b) Etude de la peroxydation lipidique induite
   Lors de la mesure de la peroxydation lipidique induite, la méthodologie est identique à celle précédemment décrite à l'exception de l'addition à l'homogénat du système inducteur de radicaux : Fe²⁺ - ascorbate. Les substances de référence sont le probucol et la vitamine E. Les concentrations des composés testés inhibant de 50 % (IC₅₀) la peroxydation du substrat sont calculées.

Il est apparu que les composés de formule (I) utilisés selon l'invention possèdent une activité antiperoxydante particulièrement intense puisqu'ils possèdent une activité antiperoxydante nettement plus importante que le probucol et la vitamine E, qui est l'antioxydant naturel de l'organisme humain.

Par exemple, le composé de l'exemple 1 présente une IC₅₀ inférieure à 10⁻⁷ M.

### EXEMPLE C ET D :

Les composés de l'invention sont également testés sur 2 tests permettant de déceler une activité antioxydante.

### EXEMPLE C : INHIBITION DE LA FORMATION DES DIENES CONJUGUES

1 cm³ d'émulsion d'acide linoléique est mis à incuber 30 min à 37°C en présence d'une solution de FeSO₄ (concentration finale 4 µmol/l) avec et sans le composé à tester (volume final 1,55 cm³).
Les diènes sont extraits par 8 cm³ de mélange chloroforme/méthanol (2/1).
Après centrifugation, 2 cm³ de la phase organique sous-jacente sont prélevés et agités 10 min avec une solution aqueuse à pH 2.
Après centrifugation, 0,250 cm³ de la phase organique sont prélevés et évaporés sous azote.
2 cm³ d'hexane sont alors ajoutés et la densité optique (DO) est mesurée à 233 nm.

Chaque dosage est réalisé en triple exemplaire. 4 à 5 h sont nécessaires pour réaliser une manipulation comprenant 6 déterminations (Blanc et référence Fe compris).
Le pourcentage d'inhibition de la formation de diènes conjugués est calculé par comparaison au 100 % de diènes formés avec le témoin Fe2+.
Les composés de l'invention s'avèrent être très efficaces dans l'inhibition de la formation des diènes, ce que confirme leur caractère antioxydant.
Par exemple, le composé de l'exemple 1, à la concentration de 10⁻⁵ M, inhibe la formation de diènes de 92 %.

### EXEMPLE D : PROTECTION DES HEMATIES CONTRE L'HEMOLYSE INDUITE PAR L'AAPH

Après centrifugation d'un prélèvement de sang humain, les hématies sont lavées trois fois avec du NaCl à 9 ‰ et 0,250 cm³ de culot sont mis en suspension dans 50 cm³ de NaCl à 9 ‰.

75 mM d'AAPH sous un volume de 0,750 cm³ sont mis en contact avec 1 cm³ de suspension d'hématies et 10⁻⁵ M du produit à tester sous un volume de 0,200 cm3. Un témoin sans AAPH est réalisé simultanément pour chaque produit testé, ainsi qu'un témoin hématies et un témoin AAPH sans produit à tester.

Après 30 min d'incubation sous agitation au bain-marie à 37°C, la suspension d'hématies est centrifugée 10 min à + 4° C (3 000 tours/min) et la densité optique à 403 nm du surnageant est mesurée par rapport au témoin sans AAPH.
Le pourcentage d'inhibition de l'hémolyse est calculé par comparaison au 100 % d'hémolyse obtenu avec le témoin AAPH.
Les composés de l'invention protègent de façon très significative les hématies de l'hémolyse.
Par exemple à 10-5 M, le composé de l'exemple 1 inhibe l'hémolyse de 74 %.

### EXEMPLE E : COMPOSITION PHARMACEUTIQUE : COMPRIMES

| Formule de préparation pour 1000 comprimés dosés à 50 mg. | |
|---|---|
| 8-méthoxy-thiopyrano[3,2-b]indole | 50 g |
| Amidon de blé | 15 g |
| Amidon de maïs | 15 g |
| Lactose | 65 g |
| Stéarate de magnésium | 2 g |
| Silice | 1 g |
| Hydroxypropylcellulose | 2 g |

**EXEMPLE F : COMPOSITION PHARMACEUTIQUE : COMPRIMES**

| Formule de préparation pour 1000 comprimés dosés à 50 mg. | |
|---|---|
| 3-hydroxy-10-méthyl-5,6,7,8,9,10-hexahydro-cyclohept[b]indole | 50 g |
| Amidon de blé | 15 g |
| Amidon de maïs | 15 g |
| Lactose | 65 g |
| Stéarate de magnésium | 2 g |
| Silice | 1 g |
| Hydroxypropylcellulose | 2 g |

## Revendications

1. Composés de formule (I) : 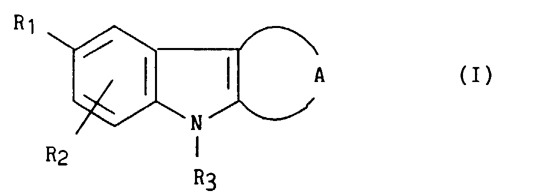 dans laquelle :
- A représente une chaîne alkylène -(CH₂)ₘ- avec m représentant 5 ou 6 ou un groupement de formule (α) 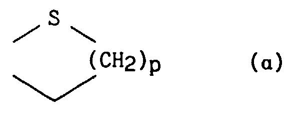 dans lequel p est compris entre 1 et 4,
- R₁ représente un radical choisi parmi :
. hydroxy,
. thiol,
. O-R₄ avec R₄ étant choisi parmi alkyle et acyle ;
. et -S-R₅ avec R₅ étant choisi parmi alkyle, phényle et benzyle ;
- R₂ représente un radical choisi parmi hydrogène, halogène, alkyle, alkoxy, et trifluorométhyle ;
- R₃ représente un radical choisi parmi alkyle, acyle, carboxyalkyle et alkoxycarbonylalkyle ; et, dans le cas où A est différent d'un pentaméthylène, R₃ peut également représenter un hydrogène,
avec la réserve que si m représente 5, R₂ représente un hydrogène et R₁ représente un méthoxy, alors R₃ ne peut pas représenter un méthyle,
leurs isomères optiques, sous forme pure ou sous forme de mélange, lorsque R₁, R₂ ou R₃ possèdent un centre chiral,
et leurs sels d'addition à une base pharmaceutiquement acceptables,
étant entendu que les termes « alkyle », « alkoxy », et « acyle » représentent des groupements linéaires ou ramifiés contenant de 1 à 6 atomes de carbone.

2. Composés selon la revendication 1 dans lesquels A représente une chaîne pentaméthylène,
leur isomères optiques sous forme pure ou sous forme de mélange,
et leurs sels d'addition à une base pharmaceutiquement acceptable.

3. Composés selon la revendication 1 dans lesquels A représente une chaîne pentaméthylène et R₁ représente un hydroxy, leurs isomères optiques sous forme pure ou sous forme de mélange,
et leurs sels d'addition à une base pharmaceutiquement acceptable.

4. Composé selon la revendication 1 qui est le 3-hydroxy-10-méthyl-5,6,7,8,9,10-hexahydro-cyclohept[b]indole et ses sels d'addition à une base pharmaceutiquement acceptable.

5. Composé selon la revendication 1 qui est le 3-méthoxy-10-méthyl-5,6,7,8,9,10,11 -heptahydrocyclooct[b]indole.

6. Composé selon la revendication 1 qui est le 8-méthoxy-thiopyrano[3,2-b]indole.

7. Composé selon la revendication 1 qui est le 2-(8-méthoxy-thiopyrano[3,2-b]indol-5-yl)acétate d'éthyle.

8. Procédé de préparation des composés de formule (I) selon la revendication 1, caractérisé en ce que l'on condense, en présence d'une base telle qu'un amidure alcalin ou sa combinaison avec un alcoolate alcalin (base complexe) et préférentiellement la base complexe NaNH₂-t.BuONa le composé de formule (II) : 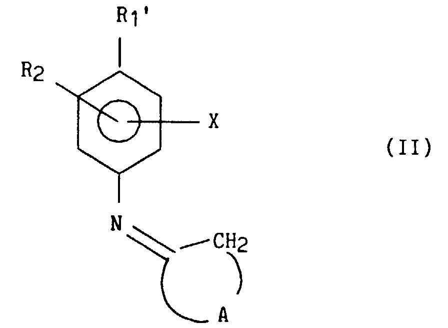 dans laquelle R₂ et A sont tels que définis dans la revendication 1, R₁' est choisi parmi -O-R₄ et -S-R₅ avec R₄ et R₅ tels que définis dans la revendication 1 et X représente un atome d'halogène, pour obtenir :
- soit un composé de formule (III) : 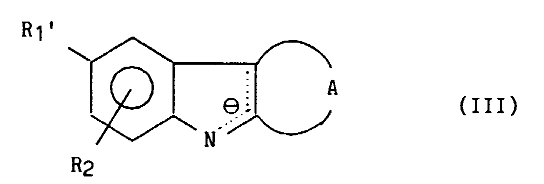 dans laquelle R₂, A et R₁' sont tels que définis précédemment,
- soit, après hydrolyse un composé de formule (Iₐ) : 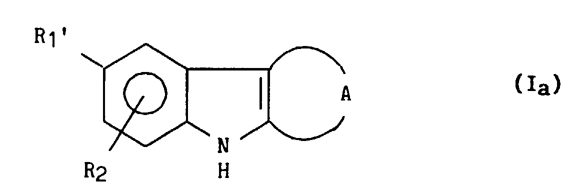 dans laquelle R₂, A et R₁' sont tels que définis précédemment,
composé de formule (III), ou composé de formule (Iₐ) sous forme de son sel alcalin, qui est substitué sur l'azote indolique par un radical de formule R₃', avec R₃' ayant la même signification que R₃ tel que défini dans la revendication 1 à l'exception de l'hydrogène, afin d'obtenir un composé de formule (Iₐ') : 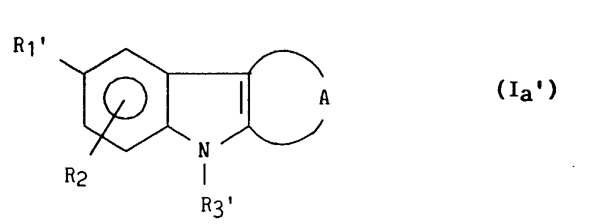 dans laquelle R₂, A, R₁' et R₃' sont tels que définis précédemment,
composés de formule (Iₐ) ou (Iₐ') qui sont, si nécessaire,
- soit soumis à une réaction de désalkylation afin d'obtenir un composé de formule (I_{b}) : 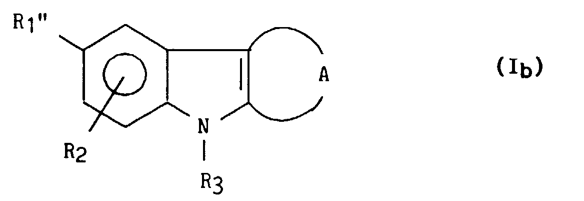 avec R₂, R₃ et A tels que définis précédemment et R₁'' représentant un groupement hydroxy ou thiol,
- soit soumis à l'action d'un mélange de formule (IV) :
Al X'₃, R₅'SH (IV)
dans laquelle X' représente un halogène et R₅' représente un alkyle, un phényle ou un benzyle,
pour obtenir, suivant les conditions d'utilisation du composé de formule (IV) :
* un composé de formule (I_{b}) tel que défini précédemment,
* ou un composé de formule (I_{c}) : 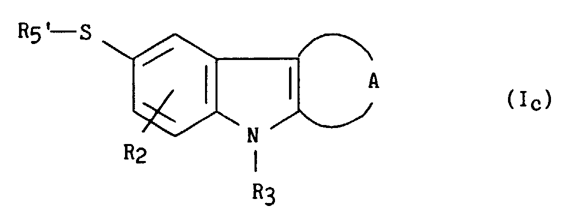 dans laquelle R₂, R₃, R₅' et A sont tels que définis précédemment,
composé de formule (I_{c}) qui peuvent être soumis à une hydrogénation pour obtenir le composé de formule (I_{d}) : 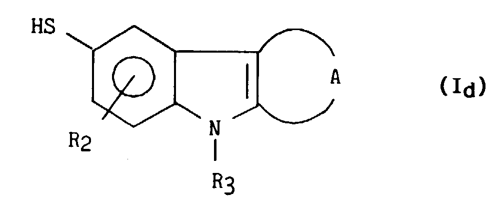 dans laquelle R₂, R₃ et A sont tels que définis précédemment,
étant entendu que les termes "alkyle", "alkoxy" et "acyle" représentent des groupements linéaires ou ramifiés contenant de 1 à 6 atomes de carbone,
les composés de formule (Iₐ), (Iₐ'), (I_{b}), (I_{c}) et (I_{d}) formant l'ensemble des composés de formule (I) selon la revendication 1,
les composés de formule (I) pouvant être, le cas échéant :
- purifiés suivant une ou plusieurs méthodes choisies parmi la cristallisation, la chromatographie sur colonne de silice, l'extraction, la filtration, et le passage sur charbon ou résine,
- séparés, sous forme pure ou sous forme de mélange, en leurs éventuels isomères optiques,
- et salifiés par une base pharmaceutiquement acceptable.

9. Compositions pharmaceutiques contenant un composé de formule (I) selon la revendication 1, ou un de ses sels d'addition à une base ou à un acide pharmaceutiquement acceptable, en combinaison avec un ou plusieurs excipients pharmaceutiquement acceptables.

10. Compositions pharmaceutiques selon la revendication 9 utiles dans les affections où intervient un phénomène de peroxydation, notamment une peroxydation lipidique.

11. Compositions pharmaceutiques selon la revendication 9 utiles dans le traitement ou la prévention des affections dues ou reliées à de tels phénomènes de peroxydation et notamment les désordres ischémiques cérébraux, rénaux ou cardiaques, l'athérosclérose, les anémies hémolytiques, les dommages dus au processus de reperfusion, et les syndromes inflammatoires.

12. Compositions pharmaceutiques selon la revendication 9 utiles dans le traitement et la prévention de l'inflammation chronique ou aiguë, articulaire, pulmonaire, cutanée, ou rénale et notamment dans la prévention et le traitement de l'arthrite, de la polyarthrite rhumatoïde, de l'ostéo-arthrose, du psoriasis, des maladies allergiques, de l'asthme, des maladies inflammatoires de l'intestin, des ulcères gastro-intestinaux,de l'ischémie, de l'athérosclérose, de la détresse respiratoire, et du choc septique.

## Patentansprüche

1. Verbindungen der Formel (I): 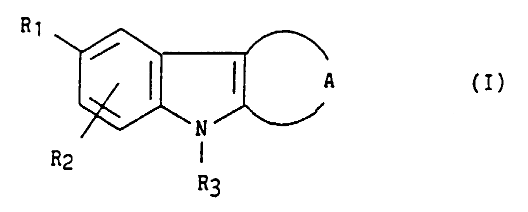 in der:
- A eine Alkylenkette -(CH₂)ₘ-, worin m 5 oder 6 darstellt, oder eine Gruppe der Formel (α) 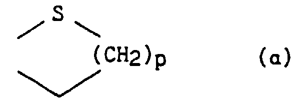 worin p einen Wert zwischen 1 und 4 besitzt,
- R₁ eine Gruppe ausgewählt aus:
. Hydroxy,
. Thiol,
. -O-R₄, worin R₄ aus Alkyl und Acyl ausgewählt ist;
. und -S-R₅, worin R₅ aus Alkyl, Phenyl und Benzyl ausgewählt ist;
- R₂ eine Gruppe ausgewählt aus Wasserstoff, Halogen, Alkyl, Alkoxy und Trifluormethyl:
- R₃ eine Gruppe ausgewählt aus Alkyl, Acyl. Carboxyalkyl und Alkoxycarbonylalkyl bedeutend wobei dann. wenn A von einer Pentamethylengruppe verschieden ist. R₃ auch ein Wasserstoffatom darstellen kann,
mit der Maßgabe, daß wenn m 5, R₂ Wasserstoff und R₁ Methoxy bedeuten, R₃ keine Methylgruppe darstellt.
deren optische Isomeren in reiner Form oder in Form einer Mischung, wenn R₁, R₂ oder R₃ ein chirales Zentrum aufweisen.
und deren Additionssalze mit einer pharmazeutisch annehmbaren Base,
wobei es sich versteht, daß die Begriffe "Alkyl", "Alkoxy" und "Acyl" für geradkettige oder verzweigte Gruppen, die 1 bis 6 Kohlenstoffatome enthalten, stehen.

2. Verbindungen nach Anspruch 1, worin A eine Pentamethylenkette darstellt.
deren optische Isomeren in reiner Form oder in Form einer Mischung und deren Additionssalze mit einer pharmazeutisch annehmbaren Base.

3. Verbindungen nach Anspruch 1, worin A eine Pentamethylenkette und R₁ eine Hydroxylgruppe bedeuten, deren optische Isomeren in reiner Form oder in Form einer Mischung und deren Additionssalze mit einer pharmazeutisch annehmbaren Base.

4. Verbindung nach Anspruch 1, nämlich 3-Hydroxy-10-methyl-5,6,7,8,9,10-hexahydro-cyclohept[b)indol und dessen Additionssalze mit einer pharmazeutisch annehmbaren Base.

5. Verbindung nach Anspruch 1, nämlich 3-Methoxy-10-methyl-5,6,7,8,9,10,11-heptahydrocyclooct[b)indol.

6. Verbindung nach Anspruch 1, nämlich 8-Methoxy-thiopyrano[3,2-b]indol.

7. Verbindung nach Anspruch 1, nämlich 2-(8-Methoxy-thiopyrano[3,2-b]indol-5-yl)-essigsäureethylester.

8. Verfahren zur Herstellung der Verbindungen der Formel (I) nach Anspruch 1, **dadurch gekennzeichnet,** daß man die Verbindung der Formel (II): 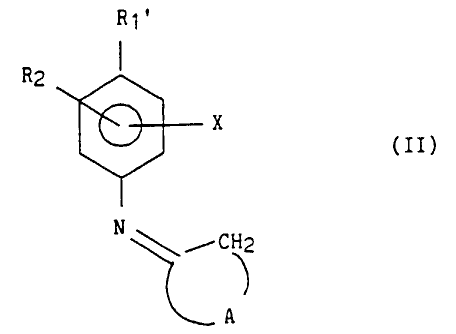 in der R₂ und A die in Anspruch 1 angegebenen Bedeutungen besitzen, R₁' aus -O-R₄ und -S-R₅ ausgewählt ist, worin R₄ und R₅ die in Anspruch 1 angegebenen Bedeutungen besitzen und X ein Halogenatom darstellt, in Gegenwart einer Base, wie eines Alkalimetallamids oder einer Kombination davon mit einem Alkalimetallalkoholat (Basenkomplex) und vorzugsweise der komplexen Base NaNH₂-tert.-Bu ONa umsetzt zur Bildung:
- entweder einer Verbindung der Formel (III): 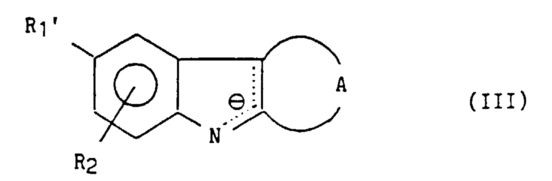 in der R₂, A und R₁' die oben angegebenen Bedeutungen besitzen,
- oder nach der Hydrolyse einer Verbindung der Formel (Iₐ): 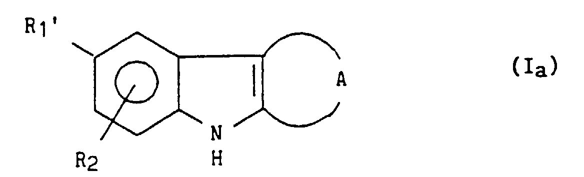 in der R₂, A und R₁' die oben angegebenen Bedeutungen besitzen,
welche Verbindung der Formel (III) oder der Formel (Iₐ) in Form ihres Alkalimetallsalzes am Stickstoffatom des Indolrings mit einer Gruppe der Formel R₃' substituiert wird. worin R₃' die gleichen Bedeutungen besitzt wie die für R₃ in Anspruch 1 angegebenen mit Ausnahme des Wasserstoffatoms, so daß man eine Verbindung der Formel (Iₐ') erhält: 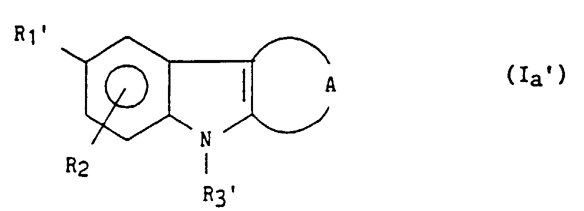 in der R₂, A, R₁' und R₃' die oben angegebenen Bedeutungen besitzen,
welche Verbindungen der Formeln (Iₐ) oder (Iₐ') erforderlichenfalls
- entweder einer Desalkylierungsreaktion unterworfen werden zur Bildung einer Verbindung der Formel (I_{b}): 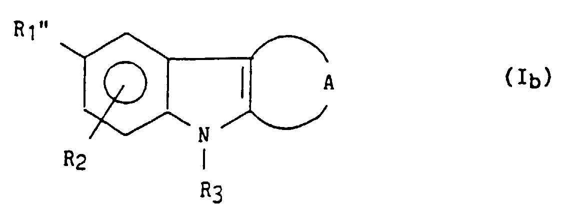 in der R₂, R₃ und A die oben angegebenen Bedeutungen besitzen und R₁ " eine Hydroxylgruppe oder eine Thiolgruppe darstellt,
- oder der Einwirkung einer Mischung der Formel (IV) unterworfen wird:
Al X'₃, R₅'SH (IV)
in der X' ein Halogenatom und R₅' eine Alkylgruppe, eine Phenylgruppe oder eine Benzylgruppe darstellen,
so daß man in Abhängigkeit von den Anwendungsbedingungen der Verbindung der Formel (IV):
* eine Verbindung der Formel (I_{b}), wie sie oben definiert worden ist,
* oder eine Verbindung der Formel (I_{c}) erhält: 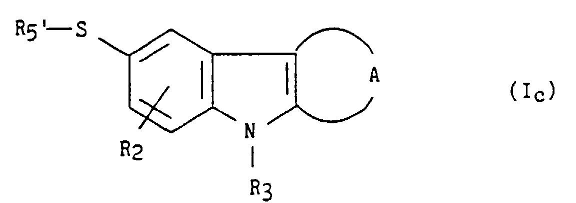 in der R₂, R₃, R₅' und A die oben angegebenen Bedeutungen besitzen,
welche Verbindung der Formel (I_{c}) einer Hydrierung unterworfen werden kann zur Bildung der Verbindung der Formel (I_{d}): 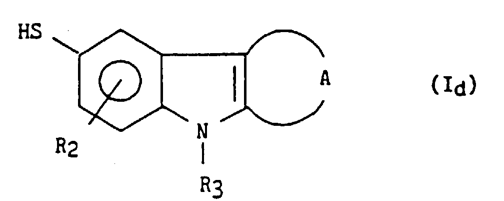 in der R₂, R₃ und A die oben angegebenen Bedeutungen besitzen, wobei es sich versteht, daß die Begriffe "Alkyl", "Alkoxy" und "Acyl" für geradkettige oder verzweigte Gruppen, die 1 bis 6 Kohlenstoffatome enthalten, stehen und
die Verbindungen der Formeln (Iₐ), (Iₐ'), (I_{b}), (I_{c}) und (I_{d}) gemeinsam die Verbindungen der Formel (I) nach Anspruch 1 bilden,
welche Verbindungen der Formel (I) gegebenenfalls:
- mit Hilfe einer oder mehrerer Methoden ausgewählt aus Kristallisation, Säulenchromatographie über Siliciumdioxid, Extraktion, Filtration und Überführung über Aktivkohle oder ein Harz gereinigt werden können.
- in ihre eventuellen optischen Isomeren in reiner Form oder in Form einer Mischung aufgetrennt werden können und
- mit einer pharmazeutisch annehmbaren Base in ihre Salze überführt werden können.

9. Pharmazeutische Zubereitungen enthaltend eine Verbindung der Formel (I) nach Anspruch 1 oder eines ihrer Additionssalze mit einer pharmazeutisch annehmbaren Base oder Säure in Kombination mit einem oder mehreren pharmazeutisch annehmbaren Trägermaterialien.

10. Pharmazeutische Zubereitungen nach Anspruch 9 zur Behandlung von Erkrankungen. bei denen ein Peroxidationsphänomen auftritt, insbesondere einer Lipidperoxidation.

11. Pharmazeutische Zubereitungen nach Anspruch 9 zur Behandlung oder der Vorbeugung von Erkrankungen. die eine Folge sind oder verknüpft sind mit solchen Peroxidationsphänomenen und insbesondere ischämischen Störungen des Gehirns. der Nieren oder des Herzens, der Atherosklerose, hämolytischen Anämien, Schäden als Folge von Reperfusionsprozessen und Entzündungszuständen.

12. Pharmazeutische Zubereitungen nach Anspruch 9 zur Behandlung und zur Vorbeugung von chronischen oder akuten Entzündungen der Gelenke, der Lungen, der Haut oder der Nieren und insbesondere bei der Vorbeutung und der Behandlung der Arthritis, der rheumatoiden Polyarthritis, der Osteoarthrose, der Psoriasis, von allergischen Erkrankungen, des Asthmas, von Entzündungserkrankungen der Eingeweide, von Magen-Dann-Geschwüren, der Ischämie, der Atherosklerose, von Atemnot und septischem Schock.

## Claims

1. Compounds of formula (I): 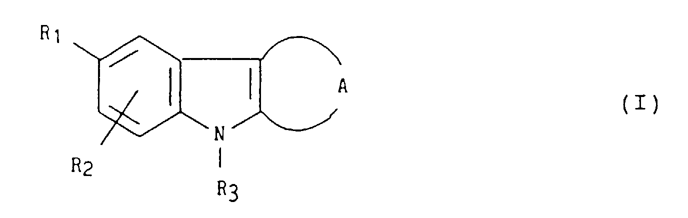 wherein:
- A represents an alkylene chain -(CH₂)ₘ- in which m is 5 or 6, or a group of the formula (α) 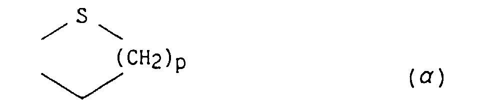 in which p is from 1 to 4,
- R₁ represents a radical selected from:
. hydroxy
. thiol
. -O-R₄ wherein R₄ is selected from alkyl and acyl;
. and -S-R₅ wherein R₅ is selected from alkyl, phenyl and benzyl;
- R₂ represents a radical selected from hydrogen, halogen, alkyl, alkoxy and trifluoromethyl;
- R₃ represents a radical selected from alkyl, acyl, carboxyalkyl and alkoxycarbonylalkyl; and, when A is other than pentamethylene, R₃ may also represent hydrogen,
with the proviso that if m is 5, R₂ is hydrogen and R₁ is methoxy, then R₃ cannot be methyl,
their optical isomers, in pure form or in the form of a mixture, when R₁, R₂ or R₃ have a chiral centre,
and their addition salts with a pharmaceutically acceptable base,
it being understood that the terms "alkyl", "alkoxy" and "acyl" represent linear or branched groups having from 1 to 6 carbon atoms.

2. Compounds according to claim 1 in which A represents a pentamethylene chain,
their optical isomers in pure form or in the form of a mixture, and the addition salts thereof with a pharmaceutically acceptable base.

3. Compounds according to claim 1 in which A represents a pentamethylene chain and R₁ represents hydroxy, their optical isomers in pure form or in the form of a mixture, and their addition salts thereof with a pharmaceutically acceptable base.

4. Compound according to claim 1 that is 3-hydroxy-10-methyl-5,6,7,8,9,10-hexahydro-cyclohept[b]indole and its addition salts with a pharmaceutically acceptable base.

5. Compound according to claim 1 that is 3-methoxy-10-methyl-5,6,7,8,9,10,11-heptahydrocyclooct[b]indole.

6. Compound according to claim 1 that is 8-methoxythiopyrano[3,2-b]indole.

7. Compound according to claim 1 that is 2-(8-methoxy-thiopyrano[3,2-b]indol-5-yl) ethyl acetate.

8. Process for the preparation of the compounds of formula (I) according to claim 1, characterised in that a compound of formula (II): 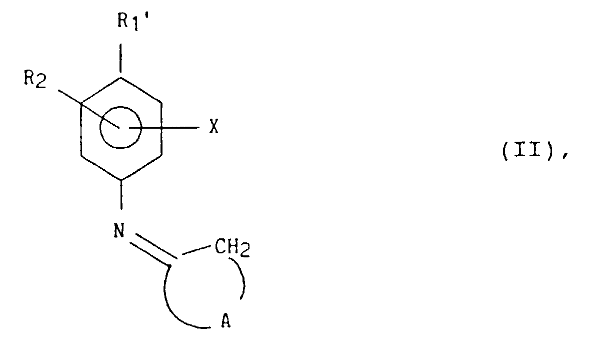 wherein R₂ and A are as defined in claim 1, R₁' is selected from -O-R₄ and -S-R₅, in which R₄ and R₅ are as defined in claim 1, and X represents a halogen atom, is condensed in the presence of a base, such as an alkali metal amide or an alkali metal amide combined with an alkali metal alcoholate (complex base), preferably the complex base NaNH₂-t.BuONa, to yield:
- either a compound of formula (III): 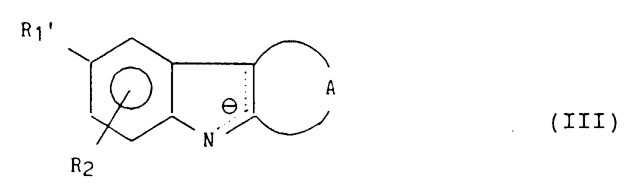 wherein R₂, A and R₁' are as defined above,
- or, after hydrolysis, a compound of formula (Iₐ): 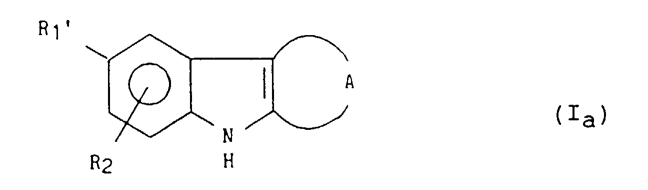 wherein R₂, A and R₁' are as defined above,
which compound of formula (III), or compound of formula (Iₐ) in the form of its alkali metal salt, is substituted on the indole nitrogen by a radical of the formula R₃' in which R₃' has the same meaning as R₃ as defined in claim 1 with the exception of hydrogen, in order to obtain a compound of formula (Iₐ'): 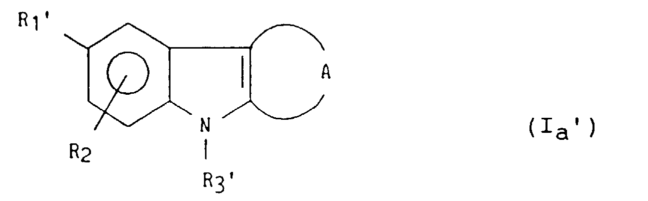 wherein R₂, A, R₁' and R₃' are as defined above,
which compounds of formulae (Iₐ) and (Iₐ') are, if necessary,
- either subjected to a dealkylation reaction in order to obtain a compound of formula (I_{b}): 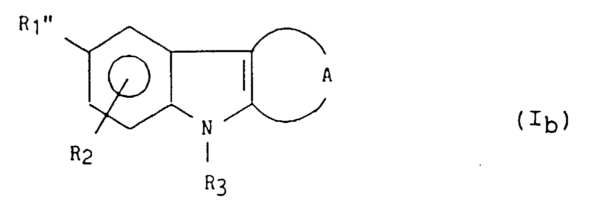 wherein R₂, R₃ and A are as defined above and R₁" represents a hydroxy or thiol group,
- or subjected to the action of a mixture of formula (IV):
Al X'₃, R₅'SH (IV)
wherein X' represents halogen and R₅' represents alkyl, phenyl or benzyl,
to obtain, depending on the conditions of use of the compound of formula (IV):
* a compound of formula (I_{b}) as defined above,
* or a compound of formula (I_{c}): 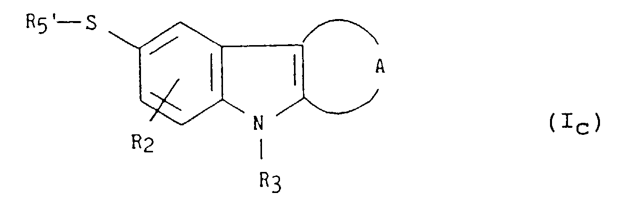 wherein R₂, R₃, R₅' and A are as defined above,
which compound of formula (Ic) may be subjected to hydrogenation to obtain a compound of formula (I_{d}): 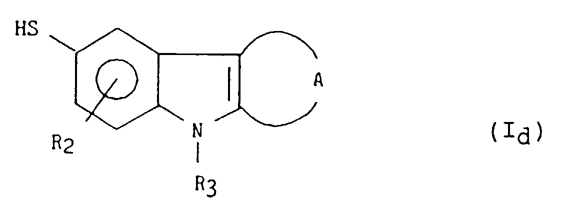 wherein R₂, R₃ and A are as defined above,
it being understood that the terms "alkyl", "alkoxy" and "acyl" represent linear or branched groups having from 1 to 6 carbon atoms,
the compounds of formula (Iₐ), (Iₐ'), (I_{b}), (I_{c}) and (I_{d}) forming the totality of the compounds of formula (I) according to claim 1,
which compounds of formula (I) may, where appropriate, be:
- purified in accordance with one or more methods selected from crystallisation, chromatography on a silica column, extraction, filtration and passage over carbon or resin,
- separated, in pure form or in the form of a mixture, into their possible optical isomers,
- and converted into their salts with a pharmaceutically acceptable base.

9. Pharmaceutical compositions comprising a compound of formula (I) according to claim 1, or an addition salt thereof with a pharmaceutically acceptable base or acid, in combination with one or more pharmaceutically acceptable excipients.

10. Pharmaceutical compositions according to claim 9 for use in disorders in which a peroxidation phenomenon, especially lipid peroxidation, is involved.

11. Pharmaceutical compositions according to claim 9 for use in the treatment or prevention of disorders due to or associated with such peroxidation phenomena, especially cerebral, renal or cardiac ischaemic disorders, atherosclerosis, haemolytic anaemia, damage due to the reperfusion process and inflammatory syndromes.

12. Pharmaceutical compositions according to claim 9 for use in the treatment and prevention of chronic or acute articular, pulmonary, cutaneous or renal inflammation, and especially in the prevention and treatment of arthritis, rheumatoid polyarthritis, osteoarthrosis, psoriasis, allergic disorders, asthma, inflammatory disorders of the intestine, gastro-intestinal ulcers, ischaemia, atherosclerosis, respiratory distress and septic shock.
